# EUROPEAN PATENT SPECIFICATION

(11) **EP 0 622 052 B1**
(45) Date of publication and mention of the grant of the patent: **29.07.1998**
(21) Application number: 94201125.5
(22) Date of filing: 23.04.1994
(51) Int. Cl.: A61C 8/00, A61C 13/00, A61F 2/28

(54) **Method for manufacturing a membrane for controlled bone regeneration**
Verfahren zur Herstellung einer Membrane für eine kontrollierte Knochenregeneration
Méthode pour la fabrication d'une membrane destiné à la régénération de l'os contrôlé

(30) Priority: 28.04.1993 BE 9300425
(43) Date of publication of application: 02.11.1994
(73) Proprietor: CEKA N.V., B-2030 Antwerpen (BE)
(72) Inventor: De Buck, Vincent, B-9100 Sint-Niklaas (BE)
(74) Representative: Donné, Eddy

(56) References cited:
- EP-A- 0 097 001
- EP-A- 0 348 061
- WO-A-91/14404
- WO-A-92/08175
- US-A- 4 684 370

## Description

The invention concerns a method for manufacturing a membrane for controlled bone regeneration at the height of a defect in the bone.

The durability and success of osteo-integrated implants, which implants are used for prosthetic reconstructions in the toothless or partly toothless upper or lower jaw, mainly depend on the quality and quantity of the bone. It is assumed that there should be at least 5 mm of bone around the implant and that the available vertical height should be at least 10 mm.

In many cases, the bone atrophy due to extraction, trauma or pathology is the cause of a malformed alveolar arch anatomy. As a result, there is not enough bone height and width to place implants. In comparison with the lower maxilla, there is more need for bone correction in the upper maxilla, because of anatomic restrictions such as the nasal passage and perinasal sinus, as well as from an aesthetic point of view, for example in the case where the patient has a high laugh line.

Autogenous as well as allogenous bone transplantations are known techniques for bone raising. These transplantations may have the shape of a horseshoe, or otherwise sections may be provided which are fixed to the bone whether or not simultaneously with the implants. Autogenous bone transplantations are disadvantageous in that the area where the bone is taken from sometimes causes more post-surgical discomforts to the patient than the implant area. Both autogenous and allogenous bone transplants have varying results. The shape of the raised alveolar arch is difficult to improve, which results in aesthetic disadvantages in the upper jaw.

EP-A-0097001 discloses a method for making an implantable prosthetic onlay, in which a digital image is made of an atrophic bone surface.

Another method for bone raising consists in the use of membranes for controlled bone generation. Such a biocompatible membrane may consist of polytetrafluoroethylene such as Gore-tex (W.L. Gore, Flagstaff, Arizona), of titanium foil or of polyurethane.

The regenerating effect with such membranes is based on the creation of a blood clot in the immediate vicinity of the required bone regeneration. The membrane encloses said blood clot and prevents the penetration of regenerating fibrous tissue in the area which is stabilized by the membrane. In a crater-shaped defect may also be provided hydroxylapatite granules. Osteogenetically active cells will form new bone under the membrane. The membrane itself may be either or not reabsorbable.

If a relatively large volume needs to be regenerated with bone, a stiff material should be used for the membrane which needs extra support, usually from an implant.

The use of a titanium implant in combination with such membranes appears to have more advantages in that the titanium surface has osteo-conductive qualities. Also, the combination of membranes with titanium may strongly advance the bone regeneration.

Up to now, such membranes are cut up during the surgery operation, so that they can be more easily reformed so as to fit up closely with the surrounding bone surface (as disclosed in WO-A-9114404, which forms the preamble of claim 1).

As a result, the useful size of both the membrane and the bone defect to be regenerated is restricted to filling up craters or bone raisings of limited size. When adjusting a membrane, one has to be very careful not to create any sharp angles while cutting and bending it. Sharp protrusions may perforate the soft tissues during the healing process.

The invention aims to remedy these disadvantages by providing a method which allows for a pre-surgical manufacture of the membrane. A membrane manufactured in this manner makes it possible to treat bigger defects and even allows for general bone raising. The membrane made according to this method fits perfectly and makes it possible to obtain the ideal volume increase of the bone. The use of such a membrane which is manufactured before the surgery makes it possible to save time and increases the patient's comfort.

The method for manufacturing a membrane for controlled bone regeneration at the height of a defect in the bone according to the invention is characterized in that a digital image is made of the bone showing the defect for which an increase in volume is required by means of a computer-controlled tomographic scanner, in that a morphologic model is made of this image on the basis of the information obtained via the scanner, in that a corresponding mould is made on the basis of said model and in that, finally, a relatively stiff foil is provided in the mould and put in the required shape with the help of the mould.

Thus, a carefully pre-shaped membrane is obtained in a quick manner which only has to be placed on the bone afterwards by means of surgery, on top of the defect. Before the membrane is applied, the cortical bone is perforated in different places so as to make sure that there is enough bleeding under the membrane. After a waiting period of for example six months, the membrane can be removed.

The image of the bone showing the defect for which an increase in volume is required can be made by scanning the defect and by processing the digital data obtained via said scanning in a digital manner.

In case of a unilateral defect, the intact side is scanned and the digital data of the intact area obtained via this scanning are digitally processed into a mirror image.

In those cases where the patient has a dental prothesis, the required increase in volume of the bone is preferably determined, however, before the bone showing the defect is scanned, the prothesis is excavated on the basal side until the surface of the excavation coincides with the perimeter of the required increase in volume, said excavation surface is covered with a contrast medium such that the perimeter of the required increase in volume is made visible by the scanner, and the outline of the contrast medium is digitally connected with the bone perimeter via digital processing of the scanner data.

The excavation of the prothesis can hereby be filled again before it is scanned and put in place again.

According to a special embodiment of the invention, a stereolithographic model is made as a model for the manufacture of the mould. According to another embodiment, a milled model is made instead.

The mould can be either positive or negative, whereby the foil can be put in the required shape by means of deep drawing, deep pressing or hydroforming. The mould can also contain a positive and a negative part, whereby the foil is put in the required shape between said parts.

According to a practical embodiment of the invention, as foil for the membrane is used a foil made of either of the following materials: titanium or polytetrafluoroethylene.

According to a preferred embodiment of the invention an implant is fixed to the bone together with the membrane.

In this embodiment, the length and the place of the implant can be exactly determined on the scanner image. The implant is then placed simultaneously with the membrane. The regenerating bone will then also osteointegrate with the implant.

In order to better explain the characteristics of the invention, some preferred embodiments of a method for manufacturing a membrane for controlled bone regeneration according to the invention are described below, as an example only without being limitative in any way, with reference to the accompanying drawings, where:
figure 1 is a schematic section of the lower jaw showing a defect of a patient with a dental prothesis, in a first stage of the method according to the invention;
figure 2 schematically represents a two-dimensional image made by a CT scanner of the lower jaw from figure 1;
figure 3 represents a section of a stereolithographic model made on the basis of the scanner images as in figure 2;
figure 4 represents a section of a mould made on the basis of the model from figure 3;
figure 5 represents a section of a membrane made on the basis of the mould from figure 4, placed on the lower jaw from figure 1;
figure 6 represents a section of the lower jaw analogous to that from figure 5, after regeneration of the bone.

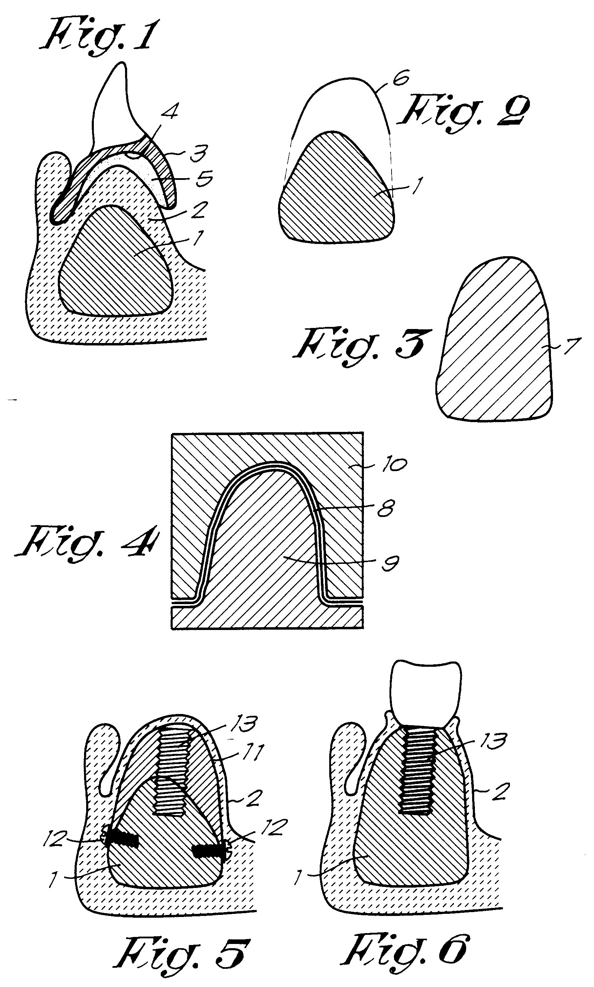

Figure 1 represents a section of the lower jaw of a patient whose bone 1, which is surrounded by the mucosa 2, shows a defect. This patient has a dental prothesis 3.

In order to make a membrane for regeneration of the bone 1, an image must be formed in the first place of the bone 1 for which an increase in volume is required. Use is made hereby of a computer-controlled tomographic scanner, also called CT scanner. The lower jaw is hereby scanned layer after layer, for example every two millimetres, by a rotating X-ray. The unidimensional information is then processed by the scanner's computer, such that a section is obtained. By means of digital image processing, a three-dimensional image can possibly be created.

Normally, the image obtained via the scanner does not take into account the required increase in volume of the bone. Since an image of the bone with the volume increase is required, said volume increase must be put in in the computer.
According to a first embodiment, said volume increase is put in via digital processing of the digital scanner data. However, adjusting the digital scanner data so as to reckon with the required volume increase of the bone is a labour-intensive and arbitrary process, except in case of a unilateral defect. In the latter case, the computer can process the scanner data of the intact area digitally into a mirror image, such that the defect side, including the volume increase, is symmetrical to the intact side.

According to another and simpler embodiment, the required volume increase of the bone is determined before the scanning takes place. Use is hereby made of the fact that the prothesis of the patient already forms a preliminary study of an ideal bone height, naturally including mucosa and tooth. The prothesis 3 is excavated on the basal side by means of milling up to about four millimetres of the outer edge. The thus obtained inner surface 4 then forms the required perimeter for the bone raising. Said surface is covered with a contrast product such as for example gutta percha or iodine.
Subsequently, the excavated space of the prothesis is filled again with a filling 5, such that the patient can support the prothesis again with the reabsorbed alveolar arch. The prothesis is placed again before proceeding to the scanning. The digital image of the scanner will now also make the outline 6 of the contrast medium visible, as represented in figure 2.

Finally, via digital processing, said outline 6 is digitally connected with the perimeter of the bone 1 as is represented by means of a dashed line in figure 2.

According to both embodiments, a digital image of the bone 1 with the required volume increase is obtained. The corresponding digital information is used to make a morphological model 7. This model may be a stereolithographic model which provides an exact copy in synthetic resin of the bone surface to be treated increased with the required increase in volume. However, also a milled model could be made.

Subsequently, on the basis of the morphological model 7 is made a mould in a low-melting alloy such as for example tin/bismuth. With the help of this mould, a relatively stiff foil 8 of bio-compatible material, preferably of titanium, is provided in the required shape which corresponds to the perimeter of the required volume increase for the bone.

The mould can be either positive or negative, and the foil 8 can be put in shape by means of deep drawing or deep pressing or a combination of both methods. The mould may contain a positive and a negative hard part, namely a round part 9 and a die 10. The die can also be made of rubber, which is better known as hydroforming. The foil 8 is put into shape between the parts 9 and 10.

The required total pressure amounts to some 1,000 bar.

As already mentioned, the use of titanium offers certain advantages. When pure titanium foil is used, titanium of degree 1 is preferably used and a foil of 0.3 mm thick.

After it has been put into shape, the foil 8 is removed from the mould 9, 10 and put on the morphological model 7 again so as to adjust the edges of the foil as a function of the contact with the bone 1.

The thus obtained foil forms the membrane 11. It is provided with small perforations on the edges so as to be able to fix titanium screws 12 to the patient's bone 1.

Before applying the membrane 11 with the patient, the outer layer of the bone is perforated in different places so as to make sure that there is enough bleeding under the membrane, which is applied immediately afterwards and fixed to the bone 1 by means of the screws 12.

It is possible to place one or several implants 13 simultaneously with the membrane 11. These implants are fixed in the bone 1 before or after the membrane is applied in the bone.

Figure 5 shows the membrane 11 with an implant 13 after they have been fixed to the bone 1. By providing an implant simultaneously with the membrane, the length and the place of said implant can be exactly determined during the scanning. The implant is then placed in the bone 1 at only a few millimetres. Around this implant 13, the regenerating bone will grow. Said bone will osteo-integrate with the implant.

After a waiting period of about six months, the membrane 11 can be removed. The bone 1 has regenerated in the meantime as represented in figure 6.

By making use of a membrane 11 which is made before the surgery, the length of the surgery operation is very short. The above-described method for the manufacture of the membrane provides a membrane which fits perfectly and which makes it possible to obtain the ideal volume increase of the bone.

The present invention is by no means limited to the above-described embodiments as represented in the drawings; on the contrary, such a method for the manufacture of a membrane for controlled bone regeneration can be made in all sorts of variants while still remaining within the scope of the invention.

In particular, the method is not restricted to the manufacture of membranes for the bone of a jaw. Membranes for bone regeneration in other places can be made according to the same method.

Also, other bio-compatible materials than titanium may be used for the relatively stiff foil, such as tetrafluorethylene or polyurethane.

Instead of stereolithography or milling, other so-called rapid prototyping or free form manufacturing techniques may be used to manufacture the morphological model, such as e.g. fused deposition modelling.

## Claims

1. Method for manufacturing a membrane (11) for controlled bone regeneration at the height of a defect in the bone (1), characterized in that a digital image is made of the bone showing the defect for which an increase in volume is required by means of a computer-controlled tomographic scanner, in that a morphologic model (7) is made of this image on the basis of the information obtained via the scanner, in that a corresponding mould (9,10) is made on the basis of said model (7) and in that, finally, a relatively stiff foil (8) is provided in the mould (9,10) and put in the required shape with the help of the mould (9,10).

2. Method according to the above claim, characterized in that the defect is scanned and in that an image of the bone (1) for which a volume increase is required is made by processing the digital data obtained via said scanning in a digital manner.

3. Method according to the above claim, characterized in that in case of a unilateral defect, the intact area is scanned and the digital data of the intact area obtained via this scanning are digitally processed into a mirror image.

4. Method according to claim 1, characterized in that in those cases where the patient has a dental prothesis (3), the required increase in volume of the bone (1) is determined before the bone showing the defect is scanned, in that the prothesis (3) is excavated on the basal side until the surface (4) of the excavation coincides with the perimeter of the required increase in volume, in that said excavation surface is covered with a contrast medium such that the perimeter of the required increase in volume is made visible by the scanner, and in that the outline (6) of the contrast medium is digitally connected with the bone perimeter via digital processing of the scanner data.

5. Method according to the above claim, characterized in that the excavation of the prothesis (3) is filled again before it is scanned and put in place again.

6. Method according to any of the above claims, characterized in that the morphological model (7) for the manufacture of the mould (9,10), is made by means of rapid prototyping or free form manufacturing techniques.

7. Method according to claim 6, characterized in that the model (7) for the manufacture of the mould (9,10) is made by stereolithography.

8. Method according to claim 6, characterized in that the model (7) for the manufacture of the mould (9,10) is made by milling.

9. Method according to any of the above claims, characterized in that as a foil (8) for the membrane (11) a foil is used made of either of the following materials: titanium, polytetrafluoroethylene or polyurethane.

## Patentansprüche

1. Verfahren zur Herstellung einer Membrane (11) für eine kontrollierte Knochenregeneration an der Stelle eines Defekts im Knochen (1), dadurch gekennzeichnet, daß, mittels eines computergesteuerten tomographischen Scanners, ein digitales Bild von dem Knochen hergestellt wird, das den Defekt zeigt, für den ein Volumenanstieg erforderlich ist, dadurch, daß von diesem Bild ein morphologisches Modell (7) hergestellt wird, auf Basis der mittels des Scanners erhaltenen Information, dadurch, daß eine entsprechende Matritze (9,10) auf Basis besagten Models hergestellt wird, und dadurch, daß, schließlich, eine relativ steife Folie (8) in der Matritze (9,10) angebracht und mit Hilfe dieser Matritze (9,10) in die erforderliche Form gebracht wird.

2. Verfahren gemaß dem obigen Anspruch, dadurch gekennzeichnet, daß der Defekt mit dem Scanner abgetastet wird und daß ein Bild des Knochens (1), für den ein Volumenanstieg erforderlich ist, hergestellt wird, indem die mittels des besagten Abtastens erhaltenen digitalen Daten auf digitale Weise bearbeitet werden.

3. Verfahren gemäß dem obigen Anspruch, dadurch gekennzeichnet, daß im Fall eines einseitigen Defekts die intakte Seite mit dem Scanner abgetastet wird und die mittels dieses Abtastens erhaltenen digitalen Daten des intakten Bereichs digital zu einem Spiegelbild verarbeitet werden.

4. Verfahren gemäß Anspruch 1, dadurch gekennzeichnet, daß in den Fällen, in denen der Patient eine Zahnprothese (3) hat, der erforderliche Volumenanstieg des Knochens (1) ermittelt wird, bevor der den Defekt aufweisende Knochen mit dem Scanner abgetastet wird, daß die Prothese (3) an der Basalseite ausgehöhlt wird, bis die Oberfläche (4) der Aushöhlung mit dem Umfang des erforderlichen Volumenanstiegs übereinstimmt, daß besagte Aushöhlungsoberfläche mit einem Kontrastmittel bedeckt wird, so daß der Umfang des erforderlichen Volumenanstiegs durch den Scanner sichtbar gemacht wird, und daß die Umrißlinie (6) des Kontrastmittels mittels digitaler Bearbeitung der Scannerdaten digital mit dem Knochenumfang verbunden wird.

5. Verfahren gemäß dem obigen Anspruch, dadurch gekennzeichnet, daß die Aushöhlung der Prothese (3) wieder gefüllt wird, bevor sie mit dem Scanner abgetastet wird, und wieder an ihren Platz gebracht wird.

6. Verfahren gemäß einem der obigen Ansprüche, dadurch gekennzeichnet, daß das morphologische Modell (7) zur Herstellung der Matritze (9,10) mittels Prototypen-Schnellfertigungs- oder Freie-Form-Herstellungstechniken gefertigt wird.

7. Verfahren gemäß Anspruch 6, dadurch gekennzeichnet, daß das Modell (7) zur Herstellung der Matritze (9,10) durch Stereolithographie gefertigt wird.

8. Verfahren gemäß Anspruch 6, dadurch gekennzeichnet, daß das Modell (7) zur Herstellung der Matritze (9,10) durch Fräsen gefertigt wird.

9. Verfahren gemäß einem der obigen Ansprüche, dadurch gekennzeichnet, daß als eine Folie (8) für die Membrane (11) eine Folie verwendet wird, die aus einem der folgenden Materialien besteht: Titan, Polytetrafluorethylen oder Polyurethan.

## Revendications

1. Procédé pour fabriquer une membrane (11) destinée à la régénération osseuse contrôlée à hauteur d'un défaut présent dans l'os (1), caractérisé en ce qu'on réalise une image numérique de l'os présentant le défaut pour lequel une augmentation du volume est requise, au moyen d'un scanner tomographique commandé par ordinateur, en ce qu'on réalise un modèle morphologique (7) de cette image sur la base des informations obtenues via le scanner, en ce qu'on réalise un moule correspondant (9, 10) sur la base dudit modèle (7) et en ce que, enfin, on insère une feuille mince (8) relativement rigide dans le moule (9, 10) et on lui donne la forme requise à l'aide du moule (9, 10).

2. Procédé selon la revendication ci-dessus, caractérisé en ce que le défaut est balayé et en ce qu'on réalise une image de l'os (1) pour lequel une augmentation du volume est requise en traitant les données numériques obtenues via ledit balayage, d'une manière numérique.

3. Procédé selon la revendication ci-dessus, caractérisé en ce que, dans le cas d'un défaut unilatéral, on balaie la zone intacte et on traite de manière numérique les données numériques de la zone intacte obtenue via ce balayage pour obtenir une image spéculaire.

4. Procédé selon la revendication 1, caractérisé en ce que, dans des cas dans lesquels le patient porte une prothèse dentaire (3), on détermine l'augmentation du volume osseux requis (1) avant de balayer l'os présentant le défaut, en ce qu'on creuse la prothèse (3) à sa base jusqu'à ce que la surface (4) du creux coïncide avec le périmètre de l'augmentation du volume requise, en ce qu'on recouvre ladite surface du creux avec un milieu de contraste de telle sorte que le périmètre de l'augmentation du volume requise est rendu visible par le scanner, et en ce qu'on relie le contour (6) du milieu de contraste de manière numérique avec le périmètre de l'os via un traitement numérique des données du scanner.

5. Procédé selon la revendication ci-dessus, caractérisé en ce qu'on remplit à nouveau le creux de la prothèse (3) avant de la balayer et de la remettre en place.

6. Procédé selon l'une quelconque des revendications ci-dessus, caractérisé en ce qu'on réalise le modèle morphologique (7) pour la fabrication du moule (9, 10) au moyen d'une technique de formation rapide d'un prototype ou d'une technique de formage libre.

7. Procédé selon la revendication 6, caractérisé en ce qu'on réalise le modèle (7) pour la fabrication du moule (9, 10) par stéréolithographie.

8. Procédé selon la revendication 6, caractérisé en ce qu'on réalise le modèle (7) pour la fabrication du moule (9, 10) par fraisage.

9. Procédé selon l'une quelconque des revendications ci-dessus, caractérisé en ce qu'on utilise, comme feuille mince (8) pour la membrane (11), une feuille mince réalisée à partir d'une quelconque des matières ci-après: du titane, du polytétrafluoréthylène ou du polyuréthanne.
